# EUROPEAN PATENT SPECIFICATION

(11) **EP 0 849 262 B1**
(45) Date of publication and mention of the grant of the patent: **17.10.2001**
(21) Application number: 97310120.7
(22) Date of filing: 15.12.1997
(51) Int. Cl.: C07D 307/32, C07C 311/08, C07C 311/14, C07D 239/42, C07D 251/42

(54) **Aminophenyl ketone derivatives and a method for the preparation thereof**
Aminophenyl-Keton-Derivate und eine Methode für ihre Herstellung
Dérivés d'aminophenyl-ketone et une méthode pour leur préparation

(30) Priority: 20.12.1996 US 771318
(43) Date of publication of application: 24.06.1998
(73) Proprietor: American Cyanamid Company, Madison, New Jersey 07940-0874 (US)
(72) Inventor: Cortés, David Andrés, Fairless Hills, Pennsylvania 19030 (US); Kremer, Kenneth Alfred Martin, Lawrenceville, New Jersey 08648 (US)
(74) Representative: Langfinger, Klaus Dieter, Dr.

(56) References cited:
- EP-A- 0 184 122
- EP-A- 0 264 467
- EP-A- 0 463 287
- EP-A- 0 655 436

## Description

The present invention provides o-aminophenyl ketone derivatives of formula I 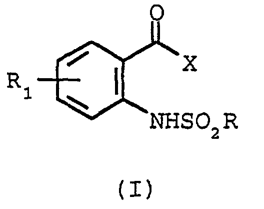 wherein R, R₁ and X are as defined below.

Compounds of formula I are useful as intermediates in the manufacture of a wide variety of herbicidal sulfamoyl urea derivatives, and in particular, the manufacture of the crop-selective herbicidal agent 1-{[o-(cyclopropyl-carbonyl)phenyl]sulfamoyl}-3-(4,6-dimethoxy-2-pyrimidinyl)urea. Also provided is a method for the preparation of said formula I intermediate.

The invention provides a compound of formula I 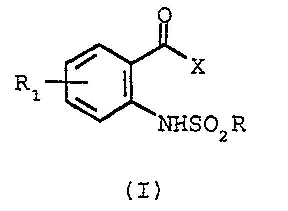 wherein
- R: is straight or branched C₁-C₆ alkyl or phenyl optionally substituted with one or more substituents the same or different selected from C₁-C₃ alkyl, C₁-C₃ alkoxy, chlorine and bromine;
- R₁: is hydrogen, cyano, nitro, halogen, formyl, C₁-C₄alkyl optionally substituted with one or more substituents the same or different selected from halogen, C₁-C₃alkoxy, C₁-C₃alkylthio, C₁-C₃₋ alkylsulfinyl and C₁-C₃alkylsulfonyl,
C₁-C₄alkoxy optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylthio optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylsulfinyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylsulfonyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylcarbonyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkoxycarbonyl optionally substituted with one or more substituents the same or different selected from halogen and C₁-C₃alkoxy,
di(C₁-C₄alkyl)amino optionally substituted with one or more substituents the same or different selected from halogen and C₁-C₃alkoxy,
di(C₁-C₄alkyl)aminocarbonyl optionally substituted with one or more substituents the same or different selected from halogen and C₁-C₃alkoxy,
di(C₁-C₄alkyl)aminosulfonyl optionally substituted with one or more substituents the same or different selected from halogen and C₁-C₃alkoxy, or
a heterocyclic ring having 2 to 6 carbon atoms and 1 to 3 heteroatoms the same or different selected from nitrogen, oxygen and sulfur atoms and being optionally substituted on the carbon atoms with one or more substituents the same or different selected from halogen, C₁-C₄alkyl and C₁C₄haloalkyl;
- X: is -(CH₂)₃-Y, cyclopropyl or tetrahydro-2-oxo-3-furyl; and
- Y: is chlorine, bromine or hydroxy; or
an acid addition salt thereof.

The invention also provides a process for preparing a compound of formula A 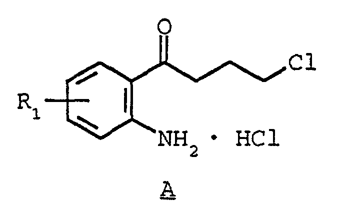 wherein R₁ is as defined in claim 1 which comprises the following steps:
i) reacting a compound of formula B 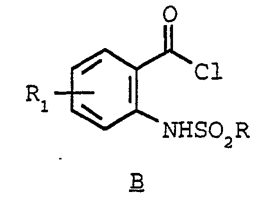 wherein R and R₁ are as defined in claim 1 with a compound of formula C 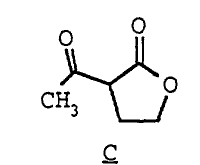 in the presence of a base and an organic solvent to form a compound of formula E 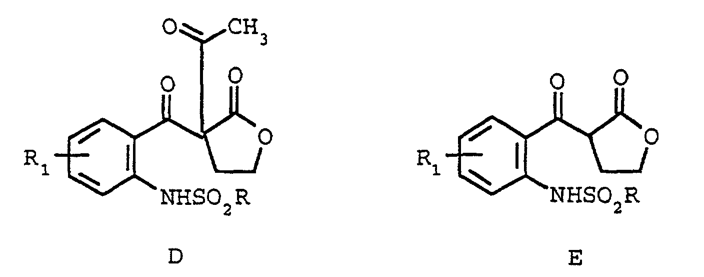 and optionally hydrolysing any compound of formula D present to give a compound of formula E; if desired isolating compound E by crystallization;
ii) reacting compound E with concentrated HCl or HBr in the presence of an organic solvent optionally in the presence of water to form a compound of formula F; 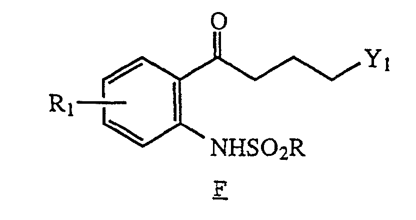 wherein Y₁ is Cl, Br or OH
iii) treating compound F with an aqueous base at an elevated temperature to give a compound of formula G; 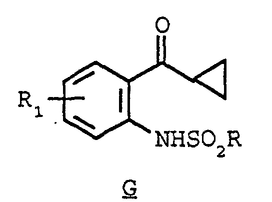
iv) treating compound G with a strong acid to give a compound of formula H; and 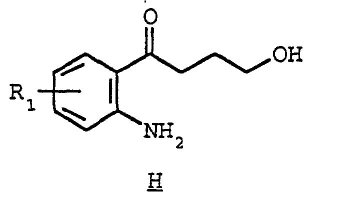 and
v) reacting compound H with HCl to form the compound of formula A.

Compound A wherein R₁ is hydrogen, 1-(o-aminophenyl)-4-chloro-1-butanone hydrochloride, is used in the manufacture of the herbicidal intermediate o-(aminophenyl)cyclopropyl ketone. A description of o-(aminophenyl)cyclopropyl ketone and its use in the manufacture of 1-{[o-(cyclopropylcarbonyl)phenyl]-sulfamoyl}-3-(4,6-dimethoxy-2-pyrimidinyl)urea herbicide is described in EP-A-0 463 287. The present invention also avoids the use of o-nitrobenzoyl chloride, which is an explosive, as an intermediate.

The base used in step (i) may be a magnesium C₁-C₄ alkoxide, preferably one which is readily available such as magnesium methoxide or magnesium ethoxide. The organic solvent used in step (i) may be an aromatic hydrocarbon or dialkyl ether such as toluene, xylene or tetrahydrofuran. The organic solvent used in step (ii) for preparing compound F may be an inert organic solvent such as toluene or xylene and the acid used in step (ii) may be a mineral acid such as concentrated HCl. When water is present compound of formula F wherein Y₁ is OH may be formed. The base used in step (iii) for preparing compound G may be an alkali metal hydroxide such as sodium hydroxide or potassium hydroxide. The elevated temperature in step (iii) may be any temperature in excess of 25 °C, preferably about 90 °-130 °C. The strong acid used in step (iv) for preparing compound H may be sulfuric acid.
The acid used for preparing compound A in step (v) may be a mineral acid such as concentrated HCl.

The compounds of the invention may be used to prepare herbicidal sulfamoyl urea compounds (K) by employing the process of the invention to prepare compounds of formula A and converting said formula A compounds to the corresponding o-(aminophenyl)cyclopropyl ketones (J) using conventional methods described in EP-A-0 655 436 and converting said phenyl ketones to the desired sulfamoylurea herbicidal products, preferably the cereal-selective, herbicidal sulfamoyl urea, 1-{(o-(cyclopropylcarbonyl)phenyl]sulfamoyl}-3-(4,6-dimethoxy-2-pyrimidinyl urea. The conversion of the phenyl ketone derivatives to sulfamoylurea herbicides may be accomplished by known processes.

The preparation is shown in flow diagram I.

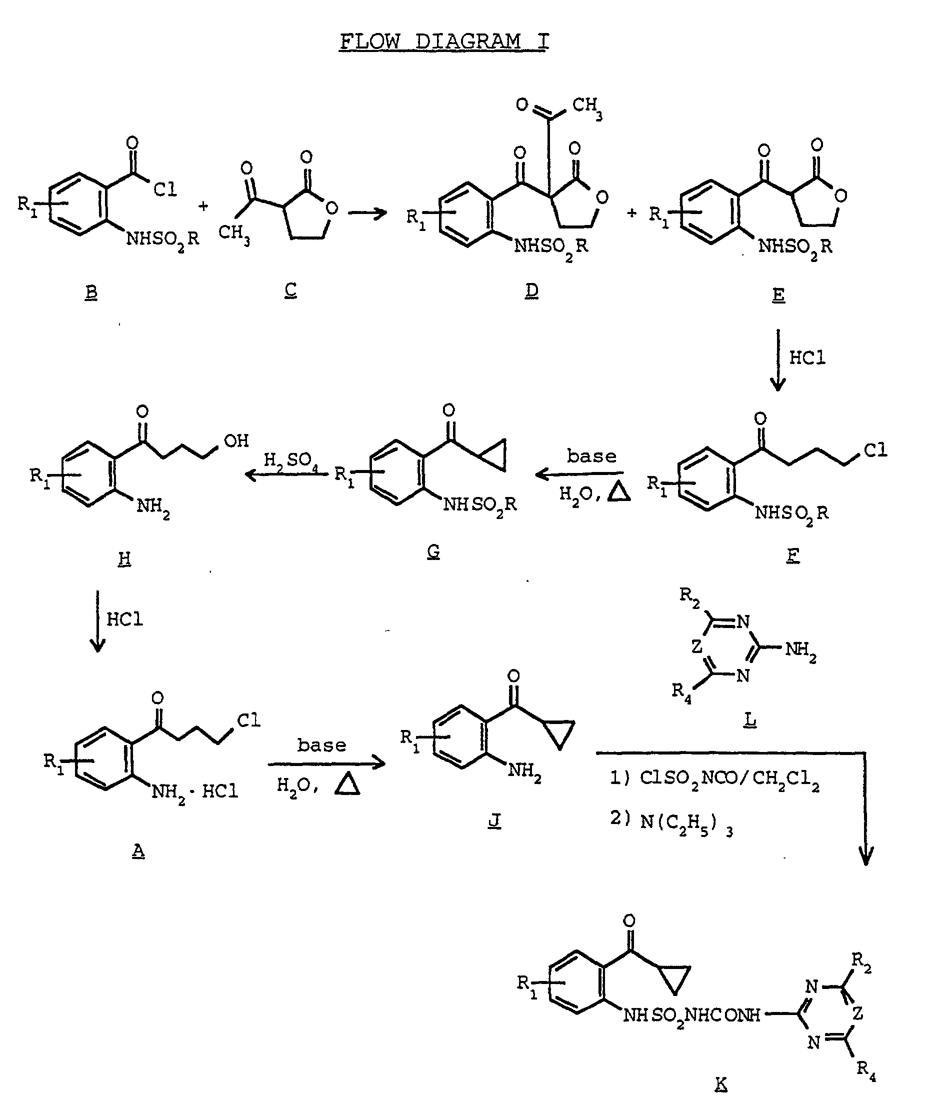

In accordance with the process of the invention, the compound of formula A is prepared as described hereinabove and converted to the o-(aminophenyl)cyclopropyl ketone compound of formula J by conventional dehydrohalogenation techniques and the formula J compound may be reacted with a 2-aminoaryl compound of formula L and chlorosulfonyl isocyanate in the presence of triethylamine and a solvent to give the desired herbicidal sulfamoyl urea of formula K.

The invention is further illustrated in the examples, below, but is not to be deemed limited thereby. The terms NMR and MS designate proton nuclear magnetic resonance and mass spectrometry, respectively.

### EXAMPLE 1

### Preparation of 2'-(Tetrahydro-2-oxo-3-furoyl)-p-toluenesulfonanilide

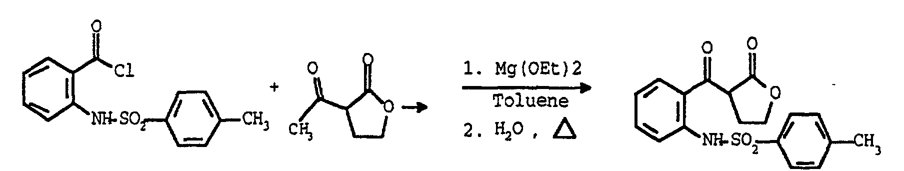

To a mixture of 40 mL of toluene and 2.03 g (178 mmol) of magnesium ethoxide in a flask under nitrogen at 5-10° C is charged 4.6 g (36 mmol) of 2-acetylbutyrolactone over 2 minutes. The resulting slurry is stirred for 10 minutes at 5-10°C and for about 1.5 hours at 20°C. The reaction mixture is treated with a solution of 10.0 g (32 mmol) of N-p-tolylsulfonyl anthranoyl chloride in 20 mL of toluene, stirred several hours at ambient temperature and for about 2 hours at 45-50°C. Water (120 mL) is added and the gray slurry is stirred for about 4 hours at 65-70°C. The pH is adjusted to 1 with concentrated sulfuric acid. The phases are separated and the organic layer is filtered to afford 7.6 g of 2'-(tetrahydro-2-oxo-3-furoyl)-p-toluenesulfonanilide. The remaining product is isolated from the organic layer filtrate by concentration in vacuo to give an additional 2.1 g of product for an overall yield of 83% (mp 138-141° C). The product is identified by NMR and MS analyses.

### EXAMPLE 2

### Preparation of 2'-(Cyclopropylcarbonyl)-p-toluenesulfonanilide

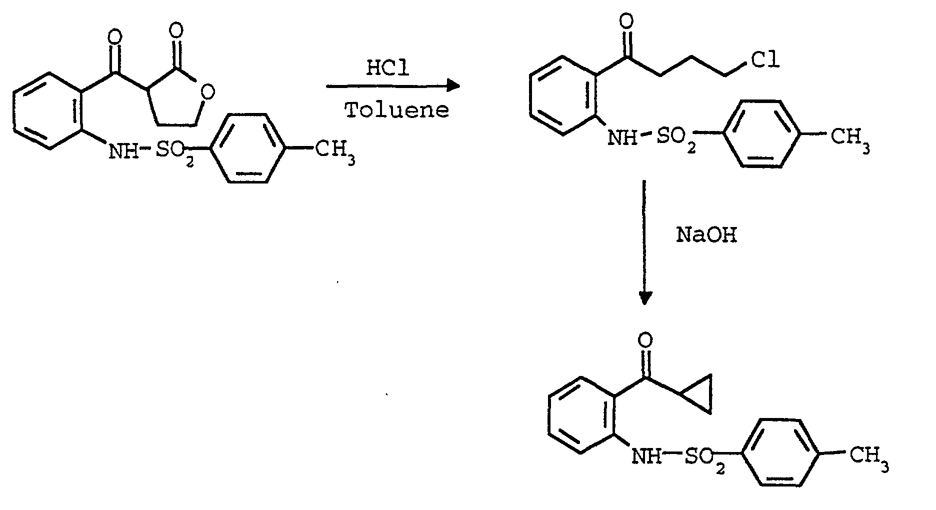

A two phase slurry mixture of 3.56 g (1.0 mmol) of the product from example 1, 25 mL of toluene and 20 mL of 37% HCl is refluxed for about 12 hours, cooled and the resulting slurry is filtered to afford 1.98 g of 4-chloro-1-(2-N-tosylaminophenyl)-1-butanone. The filtrate phases are separated and the aqueous phase is extracted with toluene. The organic phases are combined and concentrated in vacuo to give the remaining 4-chloro-1-(2-N-tosylaminophenyl)-1-butanone product (1.15 g) for an overall yield of 90% (mp 108-113 °C). The product is identified by NMR and MS analyses.

To a solution of 1.62 g (4.6 mmol) of 4-chloro-1-(2-N-tosylaminophenyl)-1-butanone in 10 mL of toluene is charged 17.3 g (28.7 mmol) of 6.6% sodium hydroxide solution. The resulting two phase mixture is refluxed for about 1 hour, cooled and adjusted to pH 1 with concentrated sulfuric acid. The organic layer is separated and concentrated in vacuo to afford 1.50 g of 2'-(cyclopropylcarbonyl)-p-toluenesulfonanilide in 100% yield (mp 92-100 °C). The product is identified by NMR and MS analyses.

### EXAMPLE 3

### Preparation of 1-(o-Aminophenyl)-4-chloro-1-butanone hydrochloride

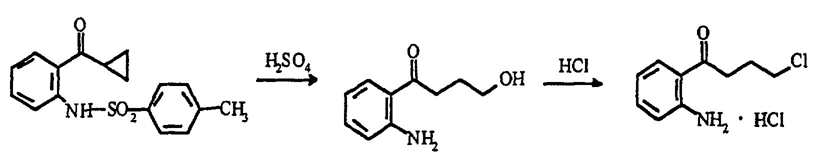

The product of example 2 (1.5 g, 4.7 mmol) is treated with 96% sulfuric acid and heated to 90 °C for 15 minutes. The solution is cooled, adjusted to pH 9 with ammonium hydroxide and extracted with methylene chloride. The combined extracts are concentrated in vacuo to provide 1-(o-aminophenyl)-4-hydroxy-1-butanone ( 80% yield, mp 58-61 °C). The product is identified by NMR and MS analyses.

A mixture of 9.3 g (5.1 mmol) of 1-(o-aminophenyl)-4-hydroxy-1-butanone, 26 mL of water and 90 mL of 37% HCl is refluxed for about 6.5 hours, cooled and filtered to afford 8.0 g of 1-(o-aminophenyl)-4-chloro-1-butanone hydrochloride. Extraction of the aqueous mother liquor with methylene chloride gives an additional 1.10 g of the title product for an overall yield of 73% (mp 142-145 °C). The product is identified by NMR and MS analyses.

### EXAMPLE 4

### Preparation of o-Aminophenyl cyclopropyl ketone

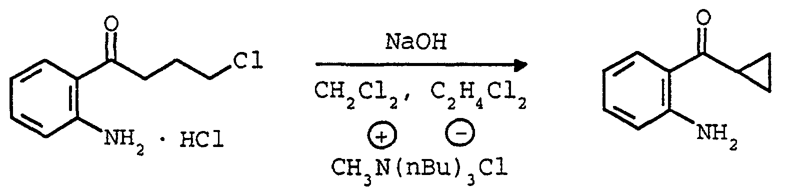

A solution of 0.30 g (1.3 mmol) of 1-(o-aminophenyl)-4-chloro-1-butanone hydrochloride in 3 mL of methylene chloride and 3 mL of ethylene dichloride is treated with 1.2 g (3 mmol) of 10% sodium hydroxide solution and 0.05 g (0.2 mmol) of 75% aqueous methyl tributylammonium chloride and heated to 50 °C for about 5 hours. After cooling to room temperature, the phases are separated. The aqueous layer is extracted with methylene chloride. The combined organic extracts are washed with water and concentrated in vacuo to give 0.14 g (70% yield) of o-aminophenyl cyclopropyl ketone (mp 46-48 °C). The product is identified by NMR and MS analyses.

## Claims

1. A compound of the formula 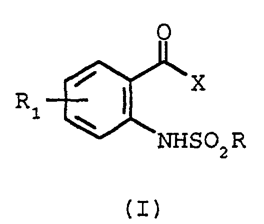 wherein
Rx is straight or branched C₁-C₆ alkyl or phenyl optionally substituted with one or more substituents the same or different selected from C₁-C₃ alkyl, C₁-C₃ alkoxy, chlorine and bromine;
R₁ is hydrogen, cyano, nitro, halogen, formyl, C₁-C₄ alkyl optionally substituted with one or more substituents the same or different selected from halogen, C₁-C₃alkoxy, C₁-C₃alkylthio, C₁-C₃-alkylsulfinyl and C₁-C₃alkylsulfonyl,
C₁-C₄alkoxy optionally substituted with one or more substituents the same or different selected from halogen, C₁-C₃alkoxy, C₁-C₃alkylthio, C₁-C₃-alkylsufinyl and C₁-C₃alkylsulfonyl,
C₁-C₄alkylthio optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylsulfinyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylsulfonyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkylcarbonyl optionally substituted with one or more substituents the same or different selected from halogen, C₁-C₃alkoxy, C₁-C₃alkylthio, C₁-C₃₋ alkylsufinyl and C₁-C₃alkylsulfonyl,
C₁-C₄alkoxycarbonyl optionally substituted by one or more substituents the same or different selected from halogen and C₁-C₃alkoxy,
di(C₁-C₄alkyl)amino optionally substituted by one or more substituents the same or different selected from halogen and C₁-C₃alkoxy,
di(C₁-C₄alkyl)aminocarbonyl optionally substituted by one or more substituents the same or different selected from halogen and C₁-C₃alkoxy,
di(C₁-C₄alkyl)aminosulfonyl optionally substituted by one or more substituents the same or different selected from halogen and C₁-C₃alkoxy, or
a heterocyclic ring having 2 to 6 carbon atoms and 1 to 3 heteroatoms the same or different selected from nitrogen, oxygen and sulfur atoms and being optionally substituted on the carbon atoms with one or more substitutents the same or different selected from halogen, C₁-C₄alkyl and C₁-C₄haloalkyl;
X is -(CH₂)₃-Y, cyclopropyl or tetrahydro-2-oxo-3-furyl;
and Y is chlorine, bromine or hydroxy;
or an acid addition salt thereof.

2. The compound according to claim 1 wherein R₁is hydrogen and optionally R is methyl or p-tolyl.

3. The compound according to claim 1 or claim 2 wherein X is cyclopropyl, -(CH₂)₃-Cl, or tetrahydro-2-oxo-3-furyl.

4. A process for preparing a compound of formula A 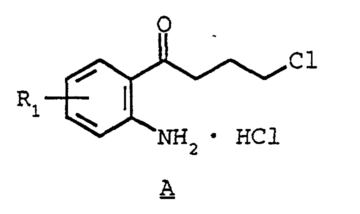 **characterized by**:
i) reacting a compound of formula B 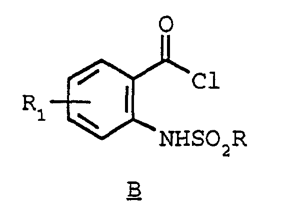 with a compound of formula C 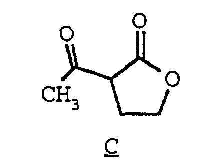 in the presence of a base and an organic solvent to form a compound of formula E 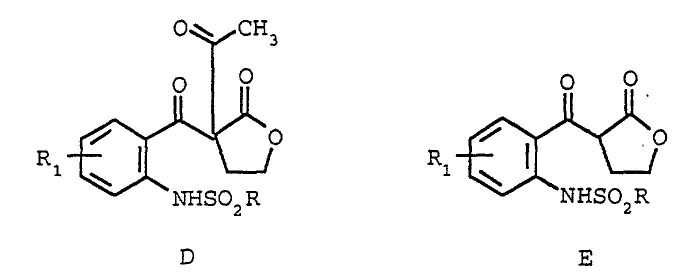 and optionally hydrolysing any compound of formula D present to give a compound of formula E;
ii) reacting compound E with concentrated HCl or HBr in the presence of an organic solvent optionally in the presence of water to form a compound of formula F 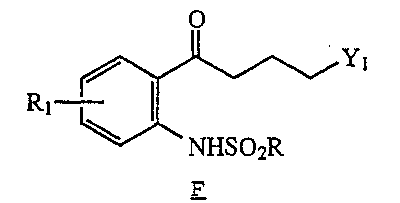 where Y₁ = Cl, Br or OH
iii) treating compound F with an aqueous base at an elevated temperature to give a compound of formula G 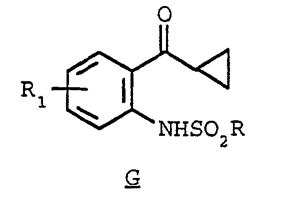
iv) treating compound G with a strong acid to give a compound of formula H 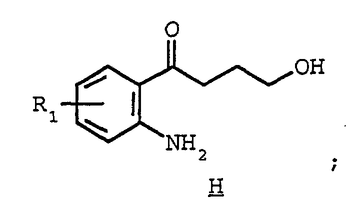 and
v) reacting compound H with HCl to form the compound of formula A;
in which formulae R and R₁ are as defined in claim 1.

5. The process according to claim 4 wherein the base in step (i) is magnesium ethoxide and the organic solvent is toluene; the organic solvent in step ii) is toluene; the base in step iii) is NaOH; and the strong acid in step iv) is sulfuric acid.

6. The process according to claim 4 or claim 5 wherein R₁ is hydrogen.

7. The process according to any one of claims 4 to 6 wherein R is p-tolyl or methyl.

8. The process according to any one of claims 4 to 7 which further comprises reacting the compound of formula A with a base to prepare o-aminophenyl cyclopropyl ketone.

9. A process for preparing a sulfamoyl urea compound of formula K 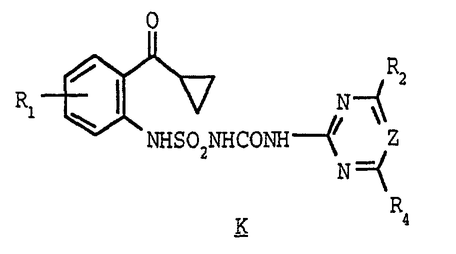 wherein R₁ is as defined in Claim 1;
z is N or CR_{3;}
R₂ is hydrogen, halogen, C₁-C₄alkyl optionally substituted with one or more substituents the same or different selected from halogen or C₁-C₃alkoxy,
C₁-C₄alkoxy optionally substituted with one or more substituents the same or different selected from halogen and C₁-C₃alkoxy,
C₁-C₄alkylthio optionally substituted with one or more substituents the same or different selected from halogen and C₁-C₃alkoxy,
C₁-C₄alkylsulfinyl optionally substituted with one or more substituents the same or different selected from halogen and C₁-C₃alkoxy,
C₁-C₄alkylsulfonyl optionally substituted with one or more the same or different selected from halogen and C₁-C₃alkoxy groups, or
C₁-C₄alkylamino or di(C₁-C₄alkyl)amino each alkyl group being optionally substituted with one or more substituents the same or different selected from halogen and C₁-C₃alkoxy;
R₃ is hydrogen or halogen; and
R₄ is hydrogen, C₁-C₄alkyl optionally substituted with one or more substituents the same or different selected from halogen and C₁-C₃alkoxy,
C₁-C₄alkoxy optionally substituted with one or more substituents the same or different selected from halogen and C₁-C₃alkoxy,
C₁-C₄alkylthio optionally substituted with one or more substituents the same or different selected from halogen and C₁-C₃alkoxy,
C₁-C₄alkylsulfinyl optionally substituted with one or more substituents the same or different selected from halogen and C₁-C₃alkoxy,
C₁-C₄alkylsulfonyl optionally substituted with one or more substituents the same or different selected from halogen and C₁-C₃alkoxy, or
C₁-C₄alkylamino or di(C₁-C₄alkyl)amino each alkyl group being optionally substituted with one or more substituents the same or different selected from halogen and C₁-C₃alkoxy,
**characterized by**:
i) preparing a compound of formula A 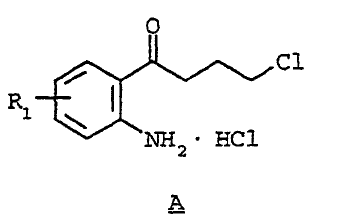 by a process as claimed in Claim 4;
ii) dehydrohalogenating the compound of formula A obtained by step (i) above to form the o-(aminophenyl)cyclopropyl ketone of formula J 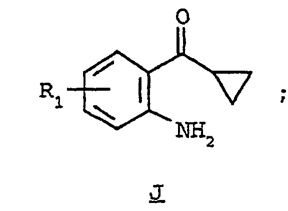 and
iii) reacting said o-(aminophenyl)cyclopropyl ketone with a 2-aminoaryl compound of formula L 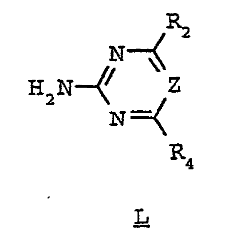 and chlorosulfonyl isocyanate, in the presence of triethylamine and a solvent to give the desired sulfamoyl urea compound of formula K.

10. The process according to claim 9 for preparing a compound of formula K wherein Z is CR₃; R₁ and R₃ are each hydrogen; and R₂ and R₄ are each methoxy.

## Patentansprüche

1. Verbindungen der Formel 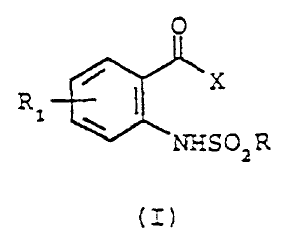 wobei
R für geradkettiges oder verzweigtes C₁-C₆-Alkyl oder Phenyl, welches gegebenenfalls durch einen oder mehrere gleiche oder verschiedene Substituenten ausgewählt aus C₁-C₃-Alkyl, C₁-C₃-Alkoxy, Chlor und Brom substituiert ist, steht;
R₁ für Wasserstoff, Cyano, Nitro, Halogen, Formyl, C₁-C₄-Alkyl, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkoxy, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkylthio, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkylsulfinyl, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkylsulfonyl, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkylcarbonyl, gegebenenfalls substituiert durch einen oder mehrere gleiche oder verschiedene Substituenten ausgewählt aus Halogen, C₁-C₃-Alkoxy, C₁-C₃-Alkylthio, C₁-C₃-Alkylsulfinyl und C₁-C₃-Alkylsulfonyl,
C₁-C₄-Alkoxycarbonyl, gegebenenfalls substituiert durch einen oder mehrere gleiche oder verschiedene Substituenten ausgewählt aus Halogen und C₁-C₃-Alkoxy,
Di(C₁-C₄-alkyl)amino, gegebenenfalls substituiert durch einen oder mehrere gleiche oder verschiedene Substituenten ausgewählt aus Halogen und C₁-C₃-Alkoxy,
Di(C₁-C₄-alkyl)aminocarbonyl, gegebenenfalls substituiert durch einen oder mehrere gleiche oder verschiedene Substituenten ausgewählt aus Halogen und C₁-C₃-Alkoxy,
Di(C₁-C₄-alkyl)aminosulfonyl, gegebenenfalls substituiert durch einen oder mehrere gleiche oder verschiedene Substituenten ausgewählt aus Halogen und C₁-C₄-Alkoxy, oder
einen heterocyclischen Ring mit 2 bis 6 Kohlenstoffatomen und 1 bis 3 gleichen oder verschiedenen Heteroatomen, ausgewählt aus Stickstoff-, Sauerstoff- und Schwefelatomen, der gegebenenfalls an den Kohlenstoffatomen durch einen oder mehrere gleiche oder verschiedene Substituenten ausgewählt aus Halogen, C₁-C₄-Alkyl und C₁-C₄-Halogenalkyl substituiert ist, steht;
X für -(CH₂)₃-Y, Cyclopropyl oder Tetrahydro-2-oxo-3-furyl steht;
und Y für Chlor, Brom oder Hydroxy steht;
und deren Säureadditionssalze.

2. Verbindungen nach Anspruch 1, wobei R₁ für Wasserstoff und R gegebenenfalls für Methyl oder p-Tolyl steht.

3. Verbindungen nach Anspruch 1 oder 2, wobei X für Cyclopropyl, -(CH₂)₃-Cl oder Tetrahydro-2-oxo-3-furyl steht.

4. Verfahren zur Darstellung von Verbindungen der Formel A 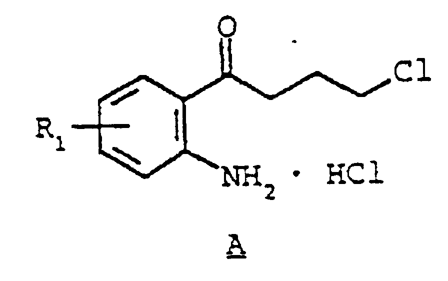 **dadurch gekennzeichnet, daß** man:
i) eine Verbindung der Formel B 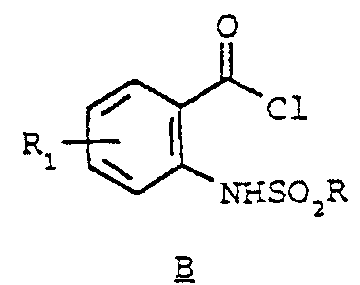 mit einer Verbindung der Formel C 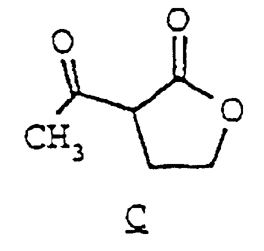 in Gegenwart einer Base und eines organischen Lösungsmittels zu einer Verbindung der Formel E 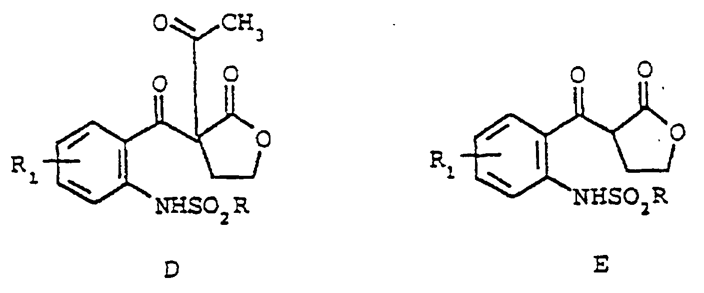 umsetzt und gegebenenfalls eine Verbindung der Formel D zu einer Verbindung der Formel E hydrolysiert;
ii) Verbindung E mit konzentrierter HCl oder HBr in Gegenwart eines organischen Lösungsmittels, gegebenenfalls in Gegenwart von Wasser, zu einer Verbindung der Formel F 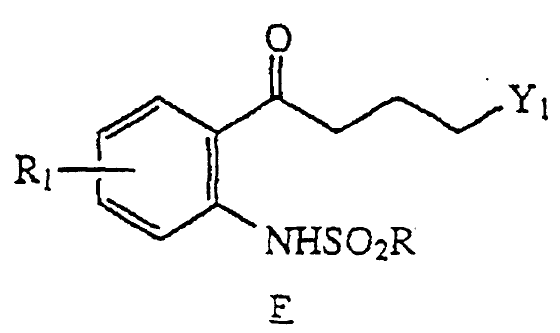 umsetzt, wobei Y₁ = Cl, Br oder OH
iii) Verbindung F bei erhöhter Temperatur mit einer wäßrigen Base behandelt und so eine Verbindung der Formel G erhält 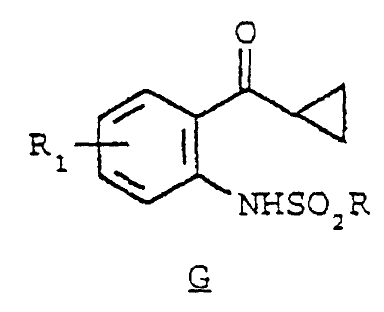
iv) Verbindung G mit einer starken Säure behandelt und so eine Verbindung der Formel H erhält 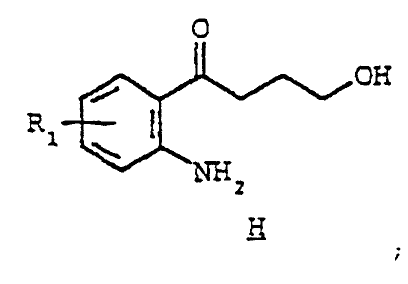 und
v) Verbindung H mit HCl zur Verbindung der Formel A umsetzt;
wobei R und R₁ in den Formeln wie in Anspruch 1 definiert sind.

5. Verfahren nach Anspruch 4, wobei es sich bei der Base in Schritt (i) um Magnesiumethanolat und bei dem organischen Lösungsmittel um Toluol; bei dem organischen Lösungsmittel in Schritt ii) um Toluol; bei der Base in Schritt iii) um NaOH; und bei der starken Säure in Schritt iv) um Schwefelsäure handelt.

6. Verfahren nach Anspruch 4 oder 5, wobei R₁ für Wasserstoff steht.

7. Verfahren nach einem der Ansprüche 4 bis 6, wobei R für p-Tolyl oder Methyl steht.

8. Verfahren nach einem der Ansprüche 4 bis 7, bei dem man weiterhin die Verbindung der Formel A mit einer Base zu o-Aminophenyl-cyclopropyl-keton umsetzt.

9. Verfahren zur Darstellung einer Sulfamoylharnstoffverbindung der Formel K 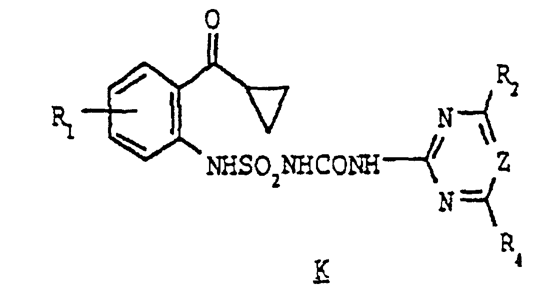 wobei R₁ wie in Anspruch 1 definiert ist;
z für N oder CR₃ steht;
R₂ für Wasserstoff, Halogen, C₁-C₄-Alkyl, gegebenenfalls substituiert durch einen oder mehrere gleiche oder verschiedene Substituenten ausgewählt aus Halogen und C₁-C₃-Alkoxy,
C₁-C₄-Alkoxy, gegebenenfalls substituiert durch einen oder mehrere gleiche oder verschiedene Substituenten ausgewählt aus Halogen und C₁-C₃-Alkoxy,
C₁-C₄-Alkylthio, gegebenfalls substituiert durch einen oder mehrere gleiche oder verschiedene Substituenten ausgewählt aus Halogen und C₁-C₃-Alkoxy,
C₁-C₄-Alkylsulfinyl, gegebenenfalls substituiert durch einen oder mehrere gleiche oder verschiedene Substituenten ausgewählt aus Halogen und C₁-C₃-Alkoxy,
C₁-C₄-Alkylsulfonyl, gegebenenfalls substituiert durch einen oder mehrere gleiche oder verschiedene Substituenten ausgewählt aus Halogen und C₁-C₃-Alkoxy, oder
C₁-C₄-Alkylamino oder Di(C₁-C₄-alkyl)amino, wobei die Alkylgruppen jeweils gegebenenfalls durch einen oder mehrere gleiche oder verschiedene Substituenten ausgewählt aus Halogen und C₁-C₃-Alkoxy substituiert sind, steht;
R₃ für Wasserstoff oder Halogen steht; und
R₄ für Wasserstoff, C₁-C₄-Alkyl, gegebenenfalls substituiert durch einen oder mehrere gleiche oder verschiedene Substituenten ausgewählt aus Halogen und C₁-C₃-Alkoxy,
C₁-C₄-Alkoxy, gegebenenfalls substituiert durch einen oder mehrere gleiche oder verschiedene Substituenten ausgewählt aus Halogen und C₁-C₃-Alkoxy,
C₁-C₄-Alkylthio, gegebenenfalls substituiert durch einen oder mehrere gleiche oder verschiedene Substituenten ausgewählt aus Halogen und C₁-C₃-Alkoxy,
C₁-C₄-Alkylsulfinyl, gegebenenfalls substituiert durch einen oder mehrere gleiche oder verschiedene Substituenten ausgewählt aus Halogen und C₁-C₃-Alkoxy,
C₁-C₄-Alkylsulfonyl, gegebenenfalls substituiert durch einen oder mehrere gleiche oder verschiedene Substituenten ausgewählt aus Halogen und C₁-C₃-Alkoxy, oder
C₁-C₄-Alkylamino oder Di(C₁-C₄-alkyl)amino, wobei die Alkylgruppen jeweils gegebenenfalls durch einen oder mehrere gleiche oder verschiedene Substituenten ausgewählt aus Halogen und C₁-C₃-Alkoxy substituiert sind, steht,
**dadurch gekennzeichnet, daß** man:
i) eine Verbindung der Formel A 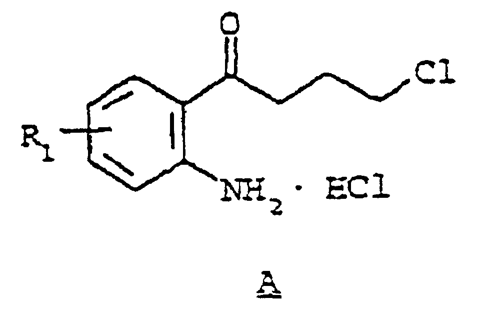 durch ein Verfahren gemäß Anspruch 4 darstellt;
ii) die oben in Schritt (i) erhaltene Verbindung der Formel A zum o-(Aminophenyl)-cyclopropyl-keton der Formel J 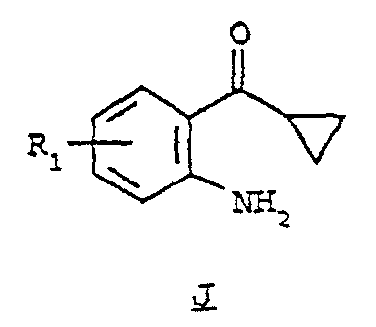 dehydrohalogeniert; und
iii) das o-(Aminophenyl)-cyclopropyl-keton mit einer 2-Aminoarylverbindung der Formel L 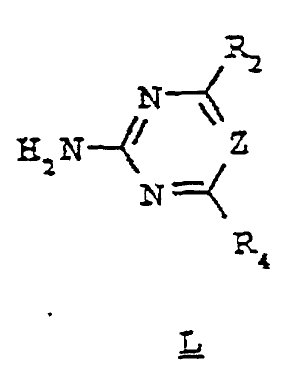 und Chlorsulfonylisocyanat in Gegenwart von Triethylamin und einem Lösungsmittel zur gewünschten Sulfamoylharnstoffverbindung der Formel K umsetzt.

10. Verfahren nach Anspruch 9 zur Darstellung einer Verbindung der Formel K, wobei Z für CR₃ steht; R₁ und R₃ jeweils für Wasserstoff stehen; und R₂ und R₄ jeweils für Methoxy stehen.

## Revendications

1. Composé de formule 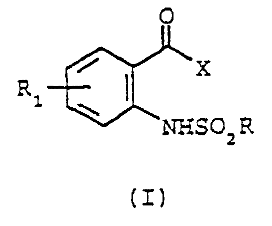 dans laquelle
R est un groupe alkyle linéaire ou ramifié en C₁-C₆ ou phényle facultativement substitué par un ou plusieurs substituants, identiques ou différents, choisis parmi les groupes alkyle en C₁-C₃, les groupes alcoxy en C₁-C₃, le chlore et le brome ;
R₁ est l'hydrogène, un groupe cyano, nitro, halogéno, formyle, alkyle en C₁-C₄ facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe alcoxy en C₁-C₄ facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe alkylthio en C₁-C₄ facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe alkylsulfinyle en C₁-C₄ facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe alkylsulfonyle en C₁-C₄ facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe (alkyle en C₁-C₄)carbonyle facultativement substitué par un ou plusieurs substituants, identiques ou différents, choisis parmi les halogènes, les groupes alcoxy en C₁-C₃, alkylthio en C₁-C₃, alkylsulfinyle en C₁-C₃ et alkylsulfonyle en C₁-C₃,
un groupe (alcoxy en C₁-C₄)carbonyle facultativement substitué par un ou plusieurs substituants, identiques ou différents, choisis parmi les halogènes et les groupes alcoxy en C₁-C₃,
un groupe di(alkyle en C₁-C₄)amino facultativement substitué par un ou plusieurs substituants, identiques ou différents, choisis parmi les halogènes et les groupes alcoxy en C₁-C₃,
un groupe di(alkyle en C₁-C₄)aminocarbonyle facultativement substitué par un ou plusieurs substituants, identiques ou différents, choisis parmi les halogènes et les groupes alcoxy en C₁-C₃,
un groupe di(alkyle en C₁-C₄)aminosulfonyle facultativement substitué par un ou plusieurs substituants, identiques ou différents, choisis parmi les halogènes et les groupes alcoxy en C₁-C₃, ou
un hétérocycle ayant 2 à 6 atomes de carbone et 1 à 3 hétéroatomes, identiques ou différents, choisis parmi les atomes d'azote, d'oxygène et de soufre, et étant facultativement substitué sur les atomes de carbone par un ou plusieurs substituants, identiques ou différents, choisis parmi les halogènes, les groupes alkyle en C₁-C₄ et halogénoalkyle en C₁-C₄ ;
X est un groupe - (CH₂)₃-Y, cyclopropyle ou tétrahydro-2-oxo-3-furyle ; et Y est le chlore, le brome ou un groupe hydroxyle ;
ou un sel d'addition d'acide de ce composé.

2. Composé selon la revendication 1, dans lequel R₁ est l'hydrogène et facultativement R est un groupe méthyle ou *p*-tolyle.

3. Composé selon la revendication 1 ou la revendication 2, dans lequel X est un groupe cyclopropyle, -(CH₂)₃-Cl ou tétrahydro-2-oxo-3-furyle.

4. Procédé pour préparer un composé de formule A 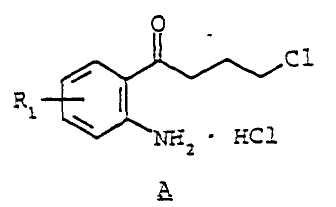 **caractérisé par** :
i) la réaction d'un composé de formule B 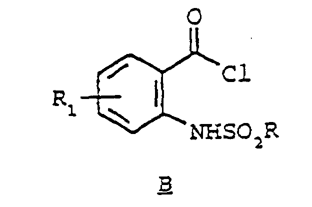 avec un composé de formule C 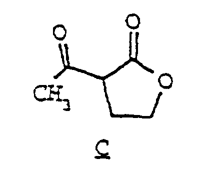 en présence d'une base et d'un solvant organique pour former un composé de formule E 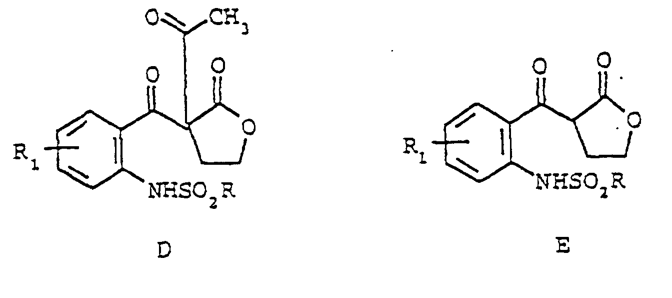 et, facultativement, l'hydrolyse de tout composé de formule D présent pour donner un composé de formule E ;
ii) la réaction du composé E avec HCl ou HBr concentré en présence d'un solvant organique, facultativement en présence d'eau, pour former un composé de formule F 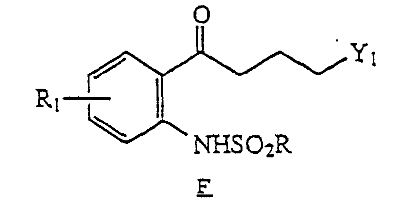 où Y₁ = Cl, Br ou OH
iii) le traitement du composé F avec une base aqueuse à une température élevée pour donner un composé de formule G 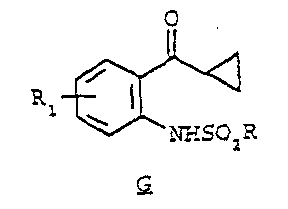
iv) le traitement du composé G avec un acide fort pour donner un composé de formule H 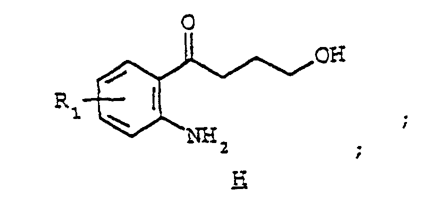 et
v) la réaction du composé H avec HCl pour former le composé de formule A ;
formules dans lesquelles R et R₁ sont tels que définis dans la revendication 1.

5. Procédé selon la revendication 4, dans lequel la base de l'étape (i) est l'éthylate de magnésium et le solvant organique est le toluène ; le solvant organique de l'étape ii) est le toluène ; la base de l'étape iii) est NaOH ; et l'acide fort de l'étape iv) est l'acide sulfurique.

6. Procédé selon la revendication 4 ou la revendication 5, dans lequel R₁ est l'hydrogène.

7. Procédé selon l'une quelconque des revendications 4 à 6, dans lequel R est un groupe *p*-tolyle ou méthyle.

8. Procédé selon l'une quelconque des revendications 4 à 7, qui comprend de plus la réaction du le composé de formule A avec une base pour préparer la *o*-aminophényl-cyclopropylcétone.

9. Procédé pour préparer une sulfamoylurée de formule K 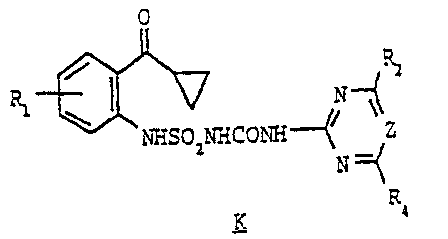 dans laquelle
R₁ est tel que défini dans la revendication 1 ;
Z est N ou CR₃ ;
R₂ est l'hydrogène, un halogène, un groupe alkyle en C₁-C₄ facultativement substitué par un ou plusieurs substituants, identiques ou différents, choisis parmi les halogènes et les groupes alcoxy en C₁-C₃,
un groupe alcoxy en C₁-C₄ facultativement substitué par un ou plusieurs substituants, identiques ou différents, choisis parmi les halogènes et les groupes alcoxy en C₁-C₃,
un groupe alkylthio en C₁-C₄ facultativement substitué par un ou plusieurs substituants, identiques ou différents, choisis parmi les halogènes et les groupes alcoxy en C₁-C₃,
un groupe alkylsulfinyle en C₁-C₄ facultativement substitué par un ou plusieurs substituants, identiques ou différents, choisis parmi les halogènes et les groupes alcoxy en C₁-C₃,
un groupe alkylsulfonyle en C₁-C₄ facultativement substitué par un ou plusieurs substituants, identiques ou différents, choisis parmi les halogènes et les groupes alcoxy en C₁-C₃, ou
un groupe alkylamino en C₁-C₄ ou di(alkyle en C₁-C₄)-amino, chaque groupe alkyle étant facultativement substitué par un ou plusieurs substituants, identiques ou différents, choisis parmi les halogènes et les groupes alcoxy en C₁-C₃ ;
R₃ est l'hydrogène ou un halogène ; et
R₄ est l'hydrogène, un groupe alkyle en C₁-C₄ facultativement substitué par un ou plusieurs substituants, identiques ou différents, choisis parmi les halogènes et les groupes alcoxy en C₁-C₃,
un groupe alcoxy en C₁-C₄ facultativement substitué par un ou plusieurs substituants, identiques ou différents, choisis parmi les halogènes et les groupes alcoxy en C₁-C₃,
un groupe alkylthio en C₁-C₄ facultativement substitué par un ou plusieurs substituants, identiques ou différents, choisis parmi les halogènes et les groupes alcoxy en C₁-C₃,
un groupe alkylsulfinyle en C₁-C₄ facultativement substitué par un ou plusieurs substituants, identiques ou différents, choisis parmi les halogènes et les groupes alcoxy en C₁-C₃,
un groupe alkylsulfonyle en C₁-C₄ facultativement substitué par un ou plusieurs substituants, identiques ou différents, choisis parmi les halogènes et les groupes alcoxy en C₁-C₃, ou
un groupe alkylamino en C₁-C₄ ou di(alkyle en C₁-C₄)-amino, chaque groupe alkyle étant facultativement substitué par un ou plusieurs substituants, identiques ou différents, choisis parmi les halogènes et les groupes alcoxy en C₁-C₃,
**caractérisé par** :
i) la préparation d'un composé de formule A 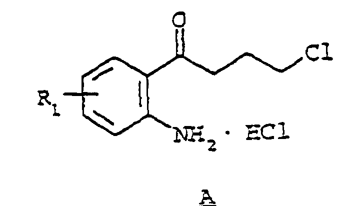 par un procédé tel que revendiqué dans la revendication 4 ;
ii) la déshydrohalogénation du composé de formule A obtenu par l'étape (i) ci-dessus pour former la *o*-(amino-phényl)cyclopropylcétone de formule J 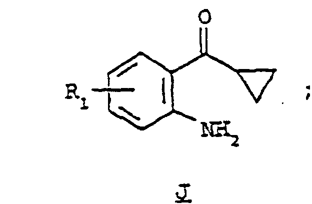 et
iii) la réaction de ladite *o*-(aminophényl)cyclopropylcétone avec un composé 2-aminoarylique de formule L 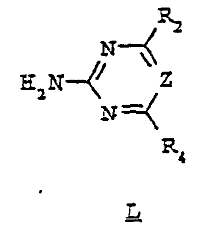 et l'isocyanate de chlorosulfonyle, en présence de triéthylamine et d'un solvant pour donner la sulfamoylurée désirée de formule K.

10. Procédé selon la revendication 9, pour préparer un composé de formule K dans lequel Z est CR₃ ; R₁ et R₃ sont chacun l'hydrogène ; et R₂ et R₄ sont chacun un groupe méthoxy.
